(19)

**Europäisches Patentamt**
**European Patent Office**
**Office européen des brevets**

(11)  **EP 3 868 875 A1**

(12)  # EUROPEAN PATENT APPLICATION

(43) Date of publication:
**25.08.2021  Bulletin 2021/34**

(21) Application number: **20158633.6**

(22) Date of filing: **20.02.2020**

(51) Int Cl.:
*C12N 7/00* (2006.01)          *C12N 15/864* (2006.01)
*C07K 14/005* (2006.01)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Medizinische Hochschule Hannover
30625 Hannover (DE)**

(72) Inventors:
• **THUM, Thomas
  30627 Hannover (DE)**

• **BÜNING, Hildegard
  30625 Hannover (DE)**
• **SANTER, Laura
  30173 Hannover (DE)**
• **BÄR, Christian
  30159 Hannover (DE)**

(74) Representative: **Taruttis, Stefan Georg
TARUTTIS Patentanwaltskanzlei
Aegidientorplatz 2b
30159 Hannover (DE)**

(54)  **VIRAL VECTOR PARTICLE BASED ON AAV2 FOR GENE THERAPY**

(57)    The invention provides a viral vector particle based on AAV2, which in its capsid protein (CAP) contains an inserted amino acid section which confers tropism for cardiomyocytes.

EP 3 868 875 A1

## Description

The present invention relates to a viral vector particle which is based on adeno-associated virus serotype 2 (AAV2) for use in gene therapy, especially for use in the treatment of cardiac diseases including cardiac defects, e.g. for use in the genetic treatment of cardiac diseases including cardiac defects. The cardiac disease or defect preferably is a disease or defect of cardiomyocytes. The cardiac disease or defect is a medical condition which is e.g. associated with cardiac overload, or cardiac insufficiency, e.g. the viral vector particle is for use in the treatment of the following medical indications: delivery of a transgene which exhibits therapeutic effects in cardiomyocytes after myocardial infarctions, cardiac hypertrophy or for the treatment of heart failure.

The viral vector particle of the invention has the advantage of being more specific for cardiomyocytes and being less efficient in transducing non-target cells, e.g. liver cells, when compared to wild-type AAV serotype 2 and 9 (AAV2 and AAV9), and to allow for expression of a transgene encoded in the viral vector particle in cardiomyocytes.

## State of the art

Perabo et al., Molecular Therapy, Vol. 8, No. 1, 151-157 (2003) describe the insertion of a random sequence of 7 amino acids into capsid proteins at position 587 referred to the VP1 capsid protein of the AAV2 virion and selection of AAV2 mutants from the human megakaryocytic cell line M-07e or B-cell chronic lymphocytic leukemia cell line Mec1 that was co-infected with adenovirus. As a result, a sequence of the mutant capsid protein was identified that conferred receptor specificity, but not cell specificity, to the viral vector.

Ying et al., Gene Therapy 17, 980-990 (2010) describe three rounds of screening of an AAV2 display peptide library for selecting vectors having higher specificity for heart tissue by injecting the AAV2 library into a mouse, isolating heart tissue slices from the mouse 3 days afterwards, and in vitro super-infecting the heart tissue slices with Ad5 in cultivation conditions. Two AAV2 variants were identified that showed increased specificity for heart tissue, but reporter gene expression from these variants was lower than expression from a wild-type AAV9 viral vector particle.

Wang et al., Nature Reviews Drug Discovery 358-378 (2019) quote that recombinant AAV (AAV vector particles or AAV vectors) in their single stranded DNA can consist of non-viral sequences flanked by the viral ITRs, so that the only viral sequences are the ITRs. The ITRs of AAV serve for genome replication and as a packaging signal during vector production.

Rockman et al., PNAS 88: 8277-8281 (1991) describe the induction of cardiac pressure overload in mice for generating a murine model for cardiac hypertrophy.

Zhang et al., Hum. Gene Ther., 1284-1296, doi: 10.1089/hu.2019/027 (2019), describe a plasmid termed pRC'99 which contains the open reading frames (ORFs) for the rep and cap proteins of AAV2 for use in cloning and generating AAV vector particles of capsid variants.

Zincarelli et al., Molecular Therapy, Vol. 16, No. 6, 1073-1080 (2008) have shown that within the naturally occurring serotypes of AAVs, AAV9 shows the highest transgene expression in heart in mice after systemic injection.

As reviewed by Hajjar & Ishikawa, Circulation Res, Vol. 120, No. 1, 33-35 (2017), the AAV9 emerged as the vector with high cardiac tropism and it thus typically used to target the heart in gene therapy research.

## Object of the invention

It is an object of the invention to provide alternative viral vector particles based on AAV2, especially capsid proteins of AAV2, which have improved specificity, also called tropism, for mouse and for human cardiomyocytes and lower affinity for liver tissue, e.g. compared to wild-type serotypes AAV2, and especially compared to AAV9. For use in gene therapy, the viral vector particle with good specificity for cardiomyocytes should allow expression of a nucleic acid coding sequence that is contained in the viral vector particle in cardiomyocytes.

## Description of the invention

The invention achieves the object by the features of the claims, and especially provides a viral vector particle based on AAV2, which in its capsid protein (CAP) C-terminally to amino acid No. 587, and/or C-terminally to amino acid No. 588, and/or C-terminally to amino acid No. 453 of the wild-type amino acid sequence of CAP contains an inserted amino acid section comprising or consisting of one of the following amino acid sequences selected from SEQ ID NO: 1 to SEQ ID NO: 53, preferably one of SEQ ID NO: 1 or SEQ ID NO: 11. The CAP of the invention, from N-terminus to C-terminus comprises or consists of the N-terminal section of CAP, which comprises or consists of amino acids 1 to 587 of the wild-type CAP, optionally a linker sequence, the inserted amino acid section, optionally a linker sequence, and the C-terminal section of CAP, which for the insertion C-terminally to amino acid No. 587 comprises or consists of amino acids 588 to 735 of the wild-type CAP, for the insertion C-terminally to amino acid No. 588 comprises or consists of amino acids 589 to 735 of the wild-type CAP, or for the insertion C-terminally to amino acid No. 453 comprises or consists of amino acids 454 to 735 of the wild-type CAP. Accordingly, the inserted amino acid section in one embodiment is inserted between amino acids N587 and R588 of the wild-type CAP amino acid sequence. In the alternative, the inserted amino acid sequence is inserted between amino acids R588 and 589 of the wild-type CAP amino acid sequence, which alternative herein is also described by the insertion between N587 and R588 and/or described by the insertion between 1456 and amino acid 454 of the wild-type CAP amino acid sequence, and all features from the description and claims apply to both the insertion between N587 and R588 of the wild-type CAP amino acid sequence, and apply to the insertion between R588 and 589, and apply to the insertion between amino acids 1453 and 454, each amino acid numbering in respect of the wild-type CAP amino acid sequence. For each of these insertion sites, especially in the embodiment in which the inserted amino acid section is inserted between amino acids No. 453 and 454 of the wild-type CAP amino acid sequence, it is preferred that the CAP amino acid sequence is additionally mutated to R585A (amino acid 585 Arg to Ala) and R588A (amino acid 588 Arg to Ala). These preferred additional mutations disturb the heparin sulfate proteoglycan binding site of wild-type CAP.

Therein, between amino acid No. 587 of CAP, or amino acid 588 of CAP, or amino acid 453 of CAP, respectively, and the N-terminal amino acid of the inserted amino acid section no additional amino acid may be present so that the inserted amino acid sequence is directly adjacent to amino acid No. 587 of CAP, respectively adjacent to amino acid No. 588 of CAP, or respectively adjacent to amino acid No. 454 of CAP, or alternatively a linker sequence of 1 to 5 amino acids, e.g. of 1 to 4 amino acids may be arranged between amino acid No. 587 of CAP, respectively amino acid No. 588 of CAP, or respectively amino acid No. 454 of CAP, and the inserted amino acid section, and/or wherein between the C-terminus of the inserted amino acid section and the remaining C-terminal portion of CAP, no additional amino acid may be present so that the inserted amino acid sequence is directly adjacent to the amino acid of the C-terminal portion of CAP, and/or a linker sequence of 1 to 4 amino acids, e.g. of 1 to 3 amino acids may be arranged between the C-terminus of the inserted amino acid section and the N-terminal amino acid of the remaining C-terminal portion of CAP.

For the insertion C-terminally to amino acid No. 587, the N-terminal amino acid of the remaining C-terminal portion of CAP preferably is amino acid No. 588 of the wild-type CAP. Preferably, the C-terminal portion of CAP, which is adjacent to the C-terminus of the inserted amino acid section, optionally with a linker sequence between the C-terminus of the inserted amino acid section and the C-terminal portion of CAP, has the amino acid sequence of amino acids No. 588 to No. 735 of SEQ ID NO: 56.

For the insertion C-terminally to amino acid No. 588, the N-terminal amino acid of the remaining C-terminal portion of CAP preferably is amino acid No. 589 of the wild-type CAP. Preferably, the C-terminal portion of CAP, which is adjacent to the C-terminus of the inserted amino acid section, optionally with a linker sequence between the C-terminus of the inserted amino acid section and the C-terminal portion of CAP, has the amino acid sequence of amino acids No. 589 to No. 735 of SEQ ID NO: 56.

For the insertion C-terminally to amino acid No. 454, the N-terminal amino acid of the remaining C-terminal portion of CAP preferably is amino acid No. 455 of the wild-type CAP. Preferably, the C-terminal portion of CAP, which is adjacent to the C-terminus of the inserted amino acid section, optionally with a linker sequence between the C-terminus of the inserted amino acid section and the C-terminal portion of CAP, has the amino acid sequence of amino acids No. 455 to No. 735 of SEQ ID NO: 56.

The inserted amino acid section can be directly adjacent to the C-terminus of amino acid No. 587, respectively directly adjacent to the C-terminus of amino acid No. 588, or respectively directly adjacent to the C-terminus of amino acid No. 455 of the wild-type amino acid sequence of CAP, or a linker sequence, e.g. of 1 to 5 amino acids, preferably of 3 amino acids, can be arranged between amino acid No. 587, respectively amino acid No. 588, or respectively amino acid No. 453 of the wild-type amino acid sequence of CAP and the inserted amino acid section. The inserted amino acid can be directly adjacent to the N-terminus of amino acid No. 588, respectively amino acid No. 589, or respectively amino acid No. 454 of the wild-type amino acid sequence of CAP, or a linker sequence, e.g. of 1 to 4 amino acids, preferably of 2 amino acids, can be arranged between the inserted amino acid section and the N-terminus of amino acid No. 588, respectively amino acid No. 589, or respectively amino acid No. 454 of the wild-type amino acid sequence of CAP. An exemplary linker sequence for arrangement between amino acid No. 588, respectively amino acid No. 589, or respectively amino acid No. 454 of the wild-type amino acid sequence of CAP and the N-terminus of the inserted amino acid section is ASA, an exemplary linker sequence for arrangement between amino acid No. 588, respectively amino acid No. 589, or respectively amino acid No. 454 of the wild-type amino acid sequence of CAP and the C-terminus of the inserted amino acid section is AA. Preferably, the inserted amino acid section together with a linker sequence at its N-terminus and a linker sequence at its C-terminus consists of 16 to 7 amino acids, e.g. 14 to 7 amino acids, more preferably of 12 amino acids.

The linker section in each case can comprise or consist of at least one of the amino acids selected from Ala, Thr, Pro, Gly, Leu, and/or Ser, which amino acids can be different from one another or all the same in each linker section.

The viral vector particles of the invention have the advantage of increased tropism, or specificity, for cardiomyocytes, e.g. in comparison to AAV2 and AAV9 having respective wild-type CAP, of reduced tropism for other cell-types than cardiomyocytes, especially reduced tropism for liver tissue, and of expression of the nucleic acid sequence that is contained in the viral vector particle in cardiomyocytes, e.g. transgene expression at a level comparable to the level of expression of the same transgene from AAV9 and much higher than the level of expression of the same transgene from AAV2 . Cardiomyocytes are part of cardiac tissue, and the viral vector particle can be for medical use, e.g. for use in the treatment of cardiac diseases or cardiac defects, e.g. for administration to a human patient. Further, the viral vector particles have the advantage of allowing its production in cells at a high titer.

Further, it was found that the viral vector particles according to the invention were neutralized to a significantly lower extent by human intravenous immunoglobulin (IVIG), which antibodies are present in the majority of persons, and which IVIG is known to neutralize wild-type AAV particles. This shows that the viral vector particles according to the invention for use in treatment have the advantage of evading neutralization by naturally occurring antibodies against AAV.

Viral vector particles containing a CAP including one of the inserted amino acid sections of SEQ ID NO: 1 to SEQ ID NO: 53, inserted between amino acid No. 587 and amino acid No. 588 of the wild-type CAP, and/or inserted between amino acid No. 588 and amino acid No. 589 of the wild-type CAP, and/or inserted between amino acid No. 453 and amino acid No. 454 of the wild-type CAP, preferably one of SEQ ID NO: 1 or SEQ ID NO: 11, have a high tropism for cardiomyocytes, allow for expression of nucleic acid sequences contained in the particles in cardiomyocytes, and significantly less expression in liver tissue, e.g. than AAV9 viral vector particles containing the same nucleic acid sequences encoding a transgene.

Accordingly, it is currently assumed that the viral particles having a CAP containing one of the inserted amino acid sections have affinity to the same cardiomyocyte surface molecule which acts as a target molecule.

The amino acid section inserted between amino acid No. 587 and amino acid No. 588 of the wild-type CAP, i.e. to the C-terminus of amino acid N. 587 of the wild-type CAP, for the preferred inserted amino acid section of SEQ ID NO: 1 is contained as an insert in the wild-type CAP in SEQ ID NO: 54, and the preferred inserted amino acid section of SEQ ID NO: 11 is contained as an insert in the wild-type CAP in SEQ ID NO: 55, wherein for each inserted amino acid section a linker sequence of amino acids ASA is arranged between the N-terminal section of the wild-type CAP, i.e. between amino acid No. 587, and a linker sequence of amino acids AA is arranged between the inserted amino acid section and the C-terminal section of CAP, i.e. between the inserted amino acid section and amino acid 588 of the wild-type CAP. The wild-type CAP is encoded by the AAV2 *cap* ORF (coding sequence is SEQ ID NO: 56). In the alternative to ASA, the linker arranged between the N-terminal section of the wild-type CAP, i.e. between amino acid No. 587 of wild-type CAP, and the inserted amino acid section, the linker can have the amino acid sequence AAA.

The viral vector particles contain a nucleic acid construct, e.g. a sense strand or an antisense strand of single-stranded DNA, comprising or consisting of an effector sequence between terminal ITR sequences of AAV2. The effector sequence can be an expression cassette encoding an effector molecule, e.g. encoding a functional non-coding RNA or a protein-coding RNA, which can be expressed from the vector construct in cardiomyocytes and thereby exhibits a therapeutic beneficial function in cardiomyocytes and thus also for the whole heart.

Herein, transduction of cardiomyocytes is the introduction of nucleic acids by the viral vector particles into cardiomyocytes, which process can also be referred to as infection by the viral vector particles, especially for use of the viral vector particles in the treatment of cardiomyocytes. Generally, the viral vector particles can be for use in the treatment of cardiomyocytes, in vivo or in vitro, e.g. for the treatment of genetic defects of cardiomyocytes, especially for transduction of cardiomyocytes.

An exemplary effector molecule can be selected from any wild-type sequence, e.g. for use in complementing a defective gene in the recipient of the viral vector particle. Exemplary effector molecules are natural genes, including genes from any species, preferably human genes.

In a specific embodiment, empty viral particles are provided, which do not contain a nucleic acid molecule. These empty viral particles can e.g. be associated with a functional molecule for delivery of the functional molecule to heart tissue. The functional molecule can e.g. be a therapeutic agent or an indicator compound, e.g. a dye or a pharmaceutically acceptable diagnostic contrast agent, or a combination of at least two of these. Empty viral particles can be produced in HEK293 cells. The process comprised the steps of transfecting the respective helper plasmid (containing the *cap* gene, if applicable with an inserted amino acid section, and the *rep* gene of wild-type AAV2) and an adenoviral helper plasmid (containing adenoviral helper functions required for AAV vector production) in HEK293 cells for AAV empty capsid production (no vector genome plasmid containing ITRs flanking the expression cassette is used in this specific case), with subsequent purification of empty capsid particles by iodixanol gradient centrifugation.

Generally, the process for producing AAV viral vector particles according to the invention can be by delivery, e.g. by plasmid transfection with or without helper virus co-infection, of all required components for AAV vector production, e.g. from a vector genome containing the transgene expression cassette flanked by ITRs, AAV rep and AAV cap genes as well as other viral helper genes necessary for AAV particle production, e.g. from adenovirus. The process can be performed in a cultivated eukaryotic host cell, followed by cell lysis and removal of cellular components and plasmid DNA, e.g. by enzymatic digestion, filtration and/or centrifugation, and further purification, e.g. by gradient density centrifugation and/or chromatography of AAV viral vector particles. The AAV viral vector particles obtained by the process comprise the capsid protein (CAP) which C-terminally to amino acid No. 587 of the wild-type amino acid sequence of CAP contains an inserted amino acid section comprising one of the amino acid sequences selected from SEQ ID NO: 1 to SEQ ID NO: 53.

The viral vector particles can be formulated for injection, e.g. for direct injection into heart tissue, or for systemic injection, e.g. intravenous (i.v.) injection.

The invention is now described by way of an example and with reference to the figures, which show in

- Fig. 1 a scheme of the selection process used for identifying CAPs with inserted amino acid sections of the invention,
- Fig. 2 shows copy numbers of viral vector particles present in different cell types of experimental animals after systemic injection,
- Fig. 3 shows transduction efficiency and specificity of viral vector particles according to the invention, and
- Fig. 4 shows results for measuring neutralisation of viral vector particles according to the invention by human intravenous immunoglobulin (IVIG).
- Fig. 5 shows results for measuring neutralisation of viral vector particles according to the invention by serum from mice which were previously injected with either AAV2, AAV9 or viral vector particles according to the invention.

## Example: Identification of AAV2 viral vector particles having specificity for cardiomyocytes

Generally, an initial library of viral particles of AAV2 capsid-mutants was generated, which contained random amino acid sections inserted into CAP. The inserted amino acid sections were encoded by nucleic acid constructs arranged in a wild-type gene encoding CAP between the sections encoding amino acids No. 587 and No. 588 of the wild-type sequence. Between the codons for amino acid 587 and for amino acid 588 of the wild-type CAP the nucleic acid constructs from 5' to 3' encoded inserted amino acid sequences consisting of the coding sequence for AAA as a linker sequence, a random 7-mer as the inserted amino acid sequence, and the coding sequence for AA as a linker sequence, resulting in the arrangement of the respective encoded amino acid sequences from N-terminus to C-terminus.

For selection of cardiomyocyte specific viral vector particles, 2 to 4 mice (male C57BL/6N, 6 to 8 weeks old) were used in which cardiac pressure overload was artificially induced as described by Rockman et al., PNAS 88: 8277-8281 (1991), which mice are also referred to as TAC-operated mice, using a 26-gauge needle. Aortic stenosis by the resultant transverse aortic constriction (TAC) was confirmed by echocardiography prior to injection of viral/vector particles. Mice were once treated by tail vein injection with $0.66 \times 10^{11}$ to $1 \times 10^{11}$ viral/vector particles.

It is assumed that the use of TAC-operated mice in the *in vivo* selection process as this mouse model supported the identification of capsid-mutant viral vector particles of AAV2 which in the CAP contained an inserted amino acid section which results in an increased tropism especially for hypertrophic cardiomyocytes. Accordingly, the viral vector particles are suitable for use in treatment of cardiac tissue, especially for targeting cardiomyocytes in the hypertrophy disease stage. At the same time, the in vivo selection in mice allows to identify capsid-mutant AAV particles which have a reduced tropism for liver and thus results in more efficient cardiomyocyte-specific transgene delivery and transgene expression, e.g. increased in comparison to wild-type AAV9. In this process, for the initial library, viral particles containing Rep and Cap genes were used, in the further selection process, Rep coding sequence was replaced by expression cassette for EGFP DNA.

As a representative transgene, the coding sequence for EGFP (enhanced green fluorescent protein) was used.

The AAV library of viral vector particles was produced from a plasmid pool by calcium phosphate transfection of HEK293 cells followed by iodixanol gradient purification of viral vector particles. Vector particle titers were determined by quantitative PCR using cap-specific or EGFP-specific primers.

The *in vivo* selection process consisted of three consecutive selection steps. In the first selection step, the initial library of viral particles was injected into mice 53 d after TAC surgery. 3 d later, cardiac tissue was fractionated to isolate cardiomyocytes. For this, mice were anaesthetized in an inhalation chamber with 4% isoflurane in oxygen. The animals were fixed in the supine position on a hot plate (at 37°C) and anesthesia was maintained with a respiratory mask (2% isoflurane in oxygen). The skin was incised and an incision was made between two tracheal trabeculae, through which a cannula was inserted. The tube was then fixed with a thread and connected to an artificial ventilation system. After disinfection of the thorax, the skin was cut along a length of 2-3 cm parallel to the rib arch, the abdomen and thorax were opened and any bleeding was dabbed. Next, the aorta was localized, lifted and gently cut. A blunt cannula was inserted through the hole and fixed with a thread. The heart was immediately retrograde perfused with pre-warmed perfusion buffer (113 mM NaCl, 4.7 mM KCl, 0.6 mM $KH_2PO_4$, 0.6 mM $Na_2HPO_4$, 1.2 mM $MgSO_4$-$7H_2O$, 0.032 mM Phenol Red, 12 mM $NaHCO_3$, 10 mM $KHCO_3$, 10 mM HEPES, 30 mM Taurine, 0.1% Glucose, 10 mM 2,3-Butanedione monoxime) for 3 min within the mouse, then removed from the mouse and perfused for additional 3 min with perfusion buffer followed by 10 min perfusion with pre-warmed digestion buffer (113 mM NaCl, 4.7 mM KCl, 0.6 mM $KH_2PO_4$, 0.6 mM $Na_2HPO_4$, 1.2 mM $MgSO_4$-$7H_2O$, 0.032 mM Phenol Red, 12 mM $NaHCO_3$, 10 mM $KHCO_3$, 10 mM HEPES, 30 mM Taurine, 0.1% Glucose, 10 mM 2,3-Butanedione monoxime, 12.5 $\mu$M $CaCl_2$, 700 U/ml Collagenase II). The atriums were removed and the ventricles were dissociated mechanically by cutting in 2.5 ml warm digestion buffer and shearing through a 1 ml syringe. Collagenase II digestion was stopped by adding 2.5 ml stop buffer (113 mM NaCl, 4.7 mM KCl, 0.6 mM $KH_2PO_4$, 0.6 mM $Na_2HPO_4$, 1.2 mM $MgSO_4$-$7H_2O$, 0.032 mM Phenol Red, 12 mM $NaHCO_3$, 10 mM $KHCO_3$, 10 mM HEPES, 30 mM Taurine, 0.1% Glucose, 10 mM 2,3-Butanedione monoxime, 12.5 $\mu$M $CaCl_2$, 10% FBS) to the cell suspension. The obtained cell suspension was filtered through a 100 $\mu$m cell strainer and the filter was washed with 1-2 ml with AMCF medium (10.8 g/l MEM HBS with NEAA (Bioconcept), 4.2 mM $NaHCO_3$, 2ng/ml vitamin B12, 1% penicillin/streptomycin (100 U/ml; 100 $\mu$g/ml), 10% FBS, pH 7.3). The appearance of rod-shaped cardiomyocytes was assessed under the microscope. Cardiomyocytes were sedimented for 10 min at room temperature (RT). The cardiomyocyte sedimentation pellet ($\rightarrow$ CMC fraction) was washed in phosphate buffered saline (PBS), centrifuged for 5 min at 900xg at 4°C, frozen in liquid nitrogen and stored at -80°C. The remaining supernatant was centrifuged for 3 min at 30xg at RT to remove residual cardiomyocytes. The cell pellet containing the residual cardiomyocytes was discarded and the supernatant containing the remaining other cardiac cell types was further processed. During the library selection, when no other specific cardiac cell types, e.g. fibroblasts or endothelial cells were required, the supernatant was centrifuged at 430xg to pellet all non-cardiomyocytes and the pellet was frozen in liquid nitrogen and stored at -80°C. If also cardiac fibroblast and endothelial cells were required (for vector copy and expression analysis of the individual vector variants), the cell pellet containing the non-myocyte fraction was instead dissolved in AMCF medium (10.8 g/l MEM HBS with NEAA (Bioconcept), 4.2 mM $NaHCO_3$, 2 ng/ml vitamin B12, 1% penicillin/streptomycin (100 U/ml; 100 $\mu$g/ml), 10% FBS, pH 7.3) and pre-plated on a 10 cm petri dish in a 1% $CO_2$ incubator for 1 h. The attached cells ($\rightarrow$ cardiac fibroblasts) were washed with PBS twice, then 2 ml PBS were added to the dish and cells were harvested with a cell scraper, centrifuged at 900xg for 5 min at 4°C. The pellet was frozen in liquid nitrogen and stored at -80°C. The supernatant of the pre-plating step, containing the non-myocyte, non-fibroblast fraction, was next centrifuged at 430xg for 5 min at 4°C. The resulting cell pellet was resuspended in 80 $\mu$l MACS buffer (MACS bovine serum albumin stock solution diluted 1:20 in auto-MACS rinsing solution, both from Miltenyi Biotec) mixed with 20 $\mu$l CD146 MACS beads (Miltenyi Biotec) and incubated for 15 min at 4°C. Afterwards, 2 ml of MACS buffer was added, the cell suspension was mixed thoroughly and centrifuged for 5 min at 430xg at 4°C. The cell pellet was resuspended in MACS buffer and transferred to a pre-washed MACS separating column. After three washing steps with MACS buffer, the separation columns were removed from the magnetic field. The EC fraction was collected by rinsing the column three times with 500 $\mu$l MACS buffer and centrifuged at 900xg for 5 min at 4°C. Pellet was frozen in liquid nitrogen and stored at -80°C.

DNA was isolated from the cell fractions using the DNeasy Blood and Tissue kit (obtained from Qiagen, Hilden, Germany) according to the manufacturer's instructions. Viral vector DNA was amplified by PCR using primers that flank the coding sequence of the inserted amino acid sequence (forward primer SEQ ID NO: 57, reverse primer SEQ ID NO: 58) for re-cloning the inserted amino acid sequences of the CAP gene of the viral particles that were accumulated in cardiomyocytes, and a secondary library of viral vector particles was generated from these re-cloned CAP gene sequences.

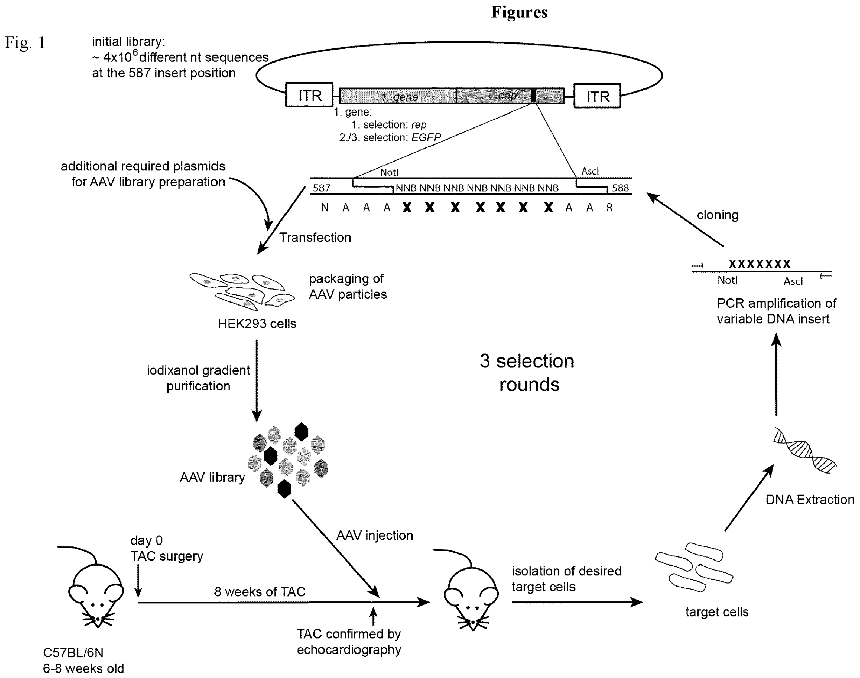

For the second selection step, in the secondary library, the *rep* gene was replaced by an expression cassette encoding EGFP, and during the production of the viral vector particles of the secondary library, the rep protein-coding sequence was supplied in *trans* by plasmid transfection. The *rep* encoding nucleotide sequence was provided on a separate plasmid which was additionally used during transfection for AAV vector production. For packaging, the vector genome of the secondary library was flanked by inverted terminal repeats (ITRs) of AAV2. The viral vector particles of the secondary library were injected 42 d after TAC surgery, and cardiomyocytes and non-myocyte cells were collected two weeks later, followed by subsequent amplification of DNA for re-cloning the nucleic acid sequences encoding the inserted amino acid sequences which were further accumulated in cardiomyocytes. The re-cloned amino acid sequences were used to generate a tertiary library which was selected the same way as the previous second selection round. The selection process is depicted in Fig. 1, wherein the target cells are cardiomyocytes, and liver tissue was analysed as the main off-target tissue of AAV vectors for evaluating specificity for cardiomyocytes.

After the three rounds of selection, the sub-library was isolated from the cardiomyocyte fraction, from the non-myocyte cardiac fraction, and from the liver tissue. The DNA of this sub-library after three selection rounds was analysed by next-generation sequencing on the 454-pyrosequencing platform (GS Junior, Roche Diagnostics), using a cap-specific primer (forward primer of SEQ ID NO: 57). Sequencing data identified coding sequences of cardiomyocyte-enriched variants that in the *cap* gene encoded inserted amino acid sequences of SEQ ID NO: 1 to SEQ ID NO: 53.

For production of individual capsid-modified vector particles which were selected from the previously conducted selection of the AAV peptide display library, oligonucleotides encoding the inserted amino acid sequences and flanking portions were used to individually generate viral vector particles having a cap protein containing one of the inserted amino acid sequences. The oligonucleotides for individual cap genes encoding one of the inserted amino acid sequences were cloned into the helper plasmid pRC'99 as described by Zhang et al., Hum. Gene Ther., 1284-1296 (2019).

The viral vector particles according to the invention which contained CAP with an inserted amino acid section of SEQ ID NO: 1 had a CAP of SEQ ID NO: 54, and the CAP with an inserted amino acid section of SEQ ID NO: 11 had a CAP of SEQ ID NO: 55.

AAV viral vector particles, both wild-type and the capsid-modified variants including a CAP with an inserted amino acid section according to the invention, and also wild-type AAV9 particles, were produced by calcium phosphate transfection of HEK293 cells, with subsequent purification of viral vector particles by iodixanol gradient purification. The individual viral vectors each containing one of the inserted amino acid sequences were produced as viral vectors encoding for EGFP under the control of the CMV promoter in a self-complementary genome conformation (scEGFP). In short, the process comprised the steps of transfecting the vector genome plasmid (CMV promoter and EGFP coding sequence, flanked by AAV2 ITRs), the respective helper plasmid (containing the *cap* gene, if applicable with an inserted amino acid section, and the *rep* gene of wild-type AAV2) and an adenoviral helper plasmid (containing adenoviral helper functions required for AAV vector production) in HEK293 cells for AAV vector production, with subsequent purification of vector particles by iodixanol gradient centrifugation.

Genomic titers of vector productions were determined by quantitative PCR using primers specific for the EGFP encoding sequence.

The copy numbers of viral vector particles present in different tissues of experimental animals on the example of CAP containing inserted amino acid section of SEQ ID NO: 1 or SEQ ID NO: 11 show that the viral vector particles containing a CAP according to the invention have higher specificity for cardiac myocytes, e.g. compared to wild-type AAV9, specifically in relation to liver cells.

AAV vector copy number analysis was performed by determining the absolute gene copy number of *Ptbp2* (polypyrimidine tract binding protein 2; two copies per diploid genome) and *EGFP* via qPCR in cardiac cell samples and organ tissue, respectively, using the absolute standard curve method. Multiplex TaqMan probe based qPCR detection was performed in a 384-well format using the TaqMan Fast Advance Master Mix (Thermo Fisher Scientific), the TaqMan Copy Number Assay for *EGFP* (Thermo Fisher Scientific; FAM-fluorescent labeled), *Ptbp2* primer (forward: TCTCCATTCCCTATGTTCATGC (SEQ ID NO: 59), reverse: GTTCCCGCAGAATGGTGAGGTG (SEQ ID NO: 60)) and a JOE-fluorescent labeled *Ptbp2* probe (5' [JOE]-ATGTTCCTCGGACCAACTTG-[BHQ1] 3'(SEQ ID NO: 61)). Each qPCR reaction contained a final concentration of 1x TaqMan Fast Advance Master Mix, 1x *EGFP* TaqMan Copy Number Assay, 150 nM *Ptbp2* TaqMan probe, 330 nM primer (forward and reverse) and 2 $\mu$l of DNA sample in a total volume of 10 $\mu$l. DNA samples comprehended either linearized plasmid DNA (containing one copy of *Ptbp2* and *EGFP* per plasmid) for an absolute copy number standard curve ($5\times10^5$, $5\times10^4$, $5\times10^3$, $5\times10^2$, $5\times10^1$ molecules/$\mu$l) or pre-diluted DNA with concentrations of 15 ng/$\mu$l.

The qPCR was run on a QuantStudio Real-Time PCR System (ThermoFisher Scientific) using the following protocol: initial activation at 50°C for 2 min and 95°C for 20 sec, followed by 40 cycles of denaturation at 95°C for 5 sec, primer/probe annealing and elongation at 56°C for 20 sec and detection of the fluorescence signal at 65°C for 20 sec. The vector copy number (VCN) in diploid cells was calculated by the following formula:

$$VCN = quantity(\mathit{EGFP})/quantity(\mathit{Ptbp2})x2.$$

The expression levels of EGFP, representing an effector gene, show that the viral vector particles according to the invention have high specificity of expression of the effector gene in cardiomyocytes. The level of transgene expression is comparable to the expression level of the same transgene delivered by wild-type AAV9. However, in comparison to AAV9, the vector particles according to the invention show reduced expression in liver tissue, which is the main off-target.

The expression of the exemplary transgene EGFP was analysed by quantitative reverse transcription PCR (qrtPCR) in cardiac cell types that were obtained by heart fractionation and in liver tissue which were collected 2 weeks after injection of the viral vectors. The results were generally normalized to the RNA input in relation to the transcripts of the TATA-box binding protein (Tbp). In detail, Reverse transcription of 65-500 ng total RNA was performed using the Biozym cDNA Synthesis Kit (Biozym) according to the manufacturer s instruction. In case of organ tissue samples, a second DNase digestion was performed directly prior reverse transcription by incubating 500 ng total RNA with 0.684 $\mu$l DNase (1:10 dilution, RNase-Free DNase Set (Qiagen)), 1.15 $\mu$l RDD buffer (RNase-Free DNase Set (Qiagen)) and 0.144 $\mu$l RNasin Ribocluclease Inhibitor (Promega) in a total volume of 11.5 $\mu$l for 30 min at 37°C. The reaction was stopped by adding 0.23 $\mu$l 62.5 mM EDTA and incubation for 5 min at 65°C. RNA dilutions of 11.5-11.73 $\mu$l containing

65-500 ng RNA (with or without second DNase digest) were reverse transcribed using 4 $\mu$l 5x cDNA synthesis buffer, 2 $\mu$l dNTP Mix (10 mM each), 1 $\mu$l hexamer primer (25 $\mu$M), 0.5 $\mu$l RNase inhibitor (40 U/$\mu$l) and 1 $\mu$l reverse transcriptase. The reaction mix was incubated at 30°C for 10 minutes, followed by 60 min at 55°C, and finally, the enzyme was heat inactivated at 99°C for 5 min. Prior to qPCR, cDNA samples were diluted with two volumes of nuclease-free $H_2O$ (1:3 dilution) and stored at -20°C.

qPCR measurements were performed in a 384-well format using the iQ SYBR Green Supermix (Biorad) according to the manufacturer's instructions. The reaction mix composed of 5 $\mu$l iQ SYBR Green Supermix, 0.05 $\mu$l of a ROX Reference Dye 1:50 dilution (Thermo Scientific), 0.025 $\mu$l Precision BlueTM Real-Time PCR Dye (BioRad), 0.5 $\mu$l of pre-mixed primer (10 $\mu$M forward and 10 $\mu$M reverse primer), 2.45 $\mu$l nuclease-free $H_2O$ and 2 $\mu$l of cDNA (1:3 dilution after cDNA synthesis) was mixed and the qPCR protocol was run on a ViiaTM 7 Real-Time PCR System (ThermoFisher Scientific) using the following protocol: initial activation at 95°C for 3 min, 45 cycles of denaturation at 95 °C for 15 seconds, primer annealing at 60°C for 30 seconds, elongation at 72 °C for 40 seconds, followed by the generation of a melting curve with fluorescence detection very 0.5 °C from 95 °C to 55 °C for 10 seconds to ensure amplification of a single PCR amplicon. EGFP expression in cardiac cell fractions and murine organs was analyzed by the relative standard method. The same 1:5 dilution series of pooled EGFP expression samples was included in all qPCR measurements in order to obtain relative standard values for all samples.

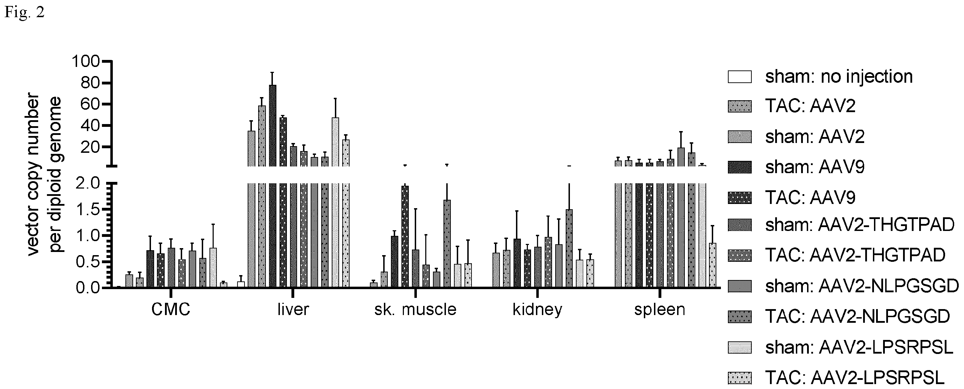

Fig. 2 shows average copy numbers (VCN) of viral vector particles in cells and organs, respectively, isolated from experimental animals, namely in cardiomyocytes (CMC), liver, skeletal muscle (sk. muscle), in kidney and spleen. The viral vector particles indicated from top to bottom are shown in the graph from left to right. The animals are indicated as sham-operated (sham) or with surgically induced transverse aortic constriction (TAC). The results show that the CAP containing the inserted amino acid section THGTPAD (SEQ ID NO: 1, AAV2-THGTPAD) and the CAP containing the inserted amino acid section NLPGSGD (SEQ ID NO: 11, AAV2-NLPGSGD) give similar copy numbers in cardiomyocytes as wild-type AAV9 (AAV9) and higher copy numbers than wild-type AAV2 (AAV2), and in liver give lower copy numbers than wild-type AAV2 and AAV9. This shows an increased specificity of CAP containing an inserted amino acid section according to the invention for cardiomyocytes over the wild-type AAV serotypes AAV2 and AAV9. In contrast, CAP with an inserted amino acid section having sequence LPSRPSL (SEQ ID NO: 62, comparative) has a lower copy number in cardiomyocytes and a higher copy number in liver, indicating a lower tropism for cardiomyocytes.

The transduction efficiency and transduction specificity of viral vector particles according to the invention were analysed by introducing viral vectors having a CAP with an inserted amino acid section of SEQ ID NO: 1 (CAP of SEQ ID NO: 54) or SEQ ID NO: 11(CAP of SEQ ID NO: 55), and for comparison wild-type AAV2 or wild-type AAV9 or a CAP containing inserted amino acid section of SEQ ID NO: 62. The viral vector particles contained the expression cassette for EGFP. The viral vector particles were injected into sham-operated mice or TAC-operated mice (each 2 to 4 animals) 6 weeks after surgery. After two weeks following the viral vector particle injection, expression of EGFP was determined by qrtPCR in cardiomyocytes, cardiac fibroblasts (CF) and cardiac endothelial cells (EC) that were fractionated from cardiac tissue, in skeletal muscle cells (sk. muscle), in liver, kidney and spleen.

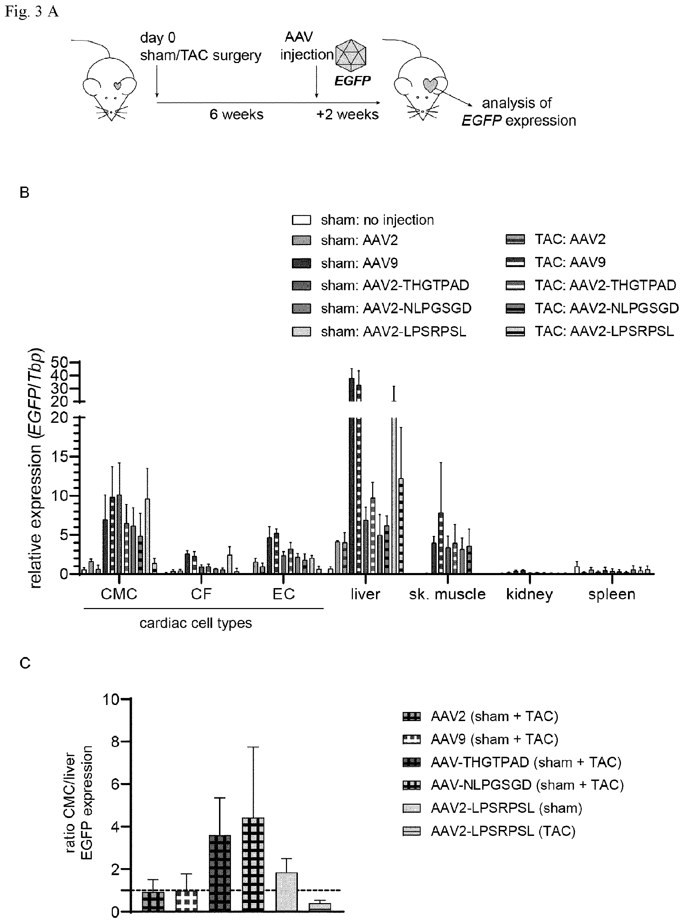

Fig. 3A schematically depicts the analytical process. Fig. 3B shows the results of detecting expression of EGFP, normalized to expression of Tbp (relative expression (EGFP/Tbp), for the sham-operated mice (sham) or TAC-operated mice (TAC) separately for each vector: wild-type AAV2 (AAV2), wild-type AAV9 (AAV9), viral vector particle with CAP containing inserted amino acid section of SEQ ID NO: 1 (AAV2-THGTPAD), viral vector particle with CAP containing inserted amino acid section of SEQ ID NO: 11 (AAV2-NLPGSGD), or comparative vector particle with CAP containing inserted amino acid section of SEQ ID NO: 62 (AAV2-LPSRPSL). These experimental combinations are indicated in Fig. 3B from top to bottom in two columns, and are indicated in this order from left to right for the different cell-types. The results show that the vector particles according to the invention in cardiac myocytes generate a higher expression of the transgene than wild-type AAV2 and a similar expression than wild-type AAV9 in cardiac myocytes of sham- and TAC-operated mice, and a lower expression of the transgene in liver, the main off-target organ, than wild-type AAV9.

Fig. 3C shows the expression of EGFP, normalized to expression of Tbp, in cardiac myocytes (CMC) relative to expression in liver, combined for the results from sham-operated animals and TAC-operated animals for the vectors AAV2, AAV9, AAV2-THGTPAC and AAV2-NLPGSGD, and separate for sham and TAC for the CAP containing comparative inserted amino acid section SEQ ID NO: 62 (AAV2-LPSRPSL). The data are given for AAV2 and AAV9 (wild type capsids), and for viral vector particles with CAP containing inserted amino acid section of SEQ ID NO: 1 (AAV2-THGTPAD), and vector with CAP containing inserted amino acid section of SEQ ID NO: 11 (AAV2-NLPGSGD), as well as for comparative vector (SEQ ID NO: 62). Based on the exemplary CAP containing inserted amino acid sections this shows that the viral vector particles on the basis of AAV2 containing CAP with an inserted amino acid section according to the invention results in significantly increased expression specificity of the effector molecule encoded by the viral vector particle in cardiac myocytes, e.g. significantly increased in comparison to wild-types AAV2 and AAV9, and in comparison to comparative CAP including inserted amino acid section of SEQ ID NO: 62.

For testing the persistence of viral vector particles according to the invention in blood serum containing human intravenous immunoglobulin (IVIG), the neutralisation activity of IVIG serum was tested. Viral vector particles containing CAP according to the invention as well as vector particles of the wild-type serotype AAV2 were incubated with different dilutions of the IVIG serum in 1 mL DMEM cell culture medium (supplemented with 10% fetal bovine serum and 1% penicillin/streptomycin). The viral vector particles were used at a concentration which in the absence of IVIG, i.e. in the cell culture medium only, transfects 30 to 40% of cells to express EGFP (30 to 40% of cells EGFP positive), which was also used as a positive control. The viral vector particles were incubated in the cell culture medium in mixture with or without IVIG serum for 1 h at room temperature and then added to HEK293 cells cultivated in 12-well plates. After an incubation of 48 hours under cell culture conditions, the HEK293 cells were analysed by fluorescence activated cell sorting (FACS) to determine the percentage of EGFP-positive cells.

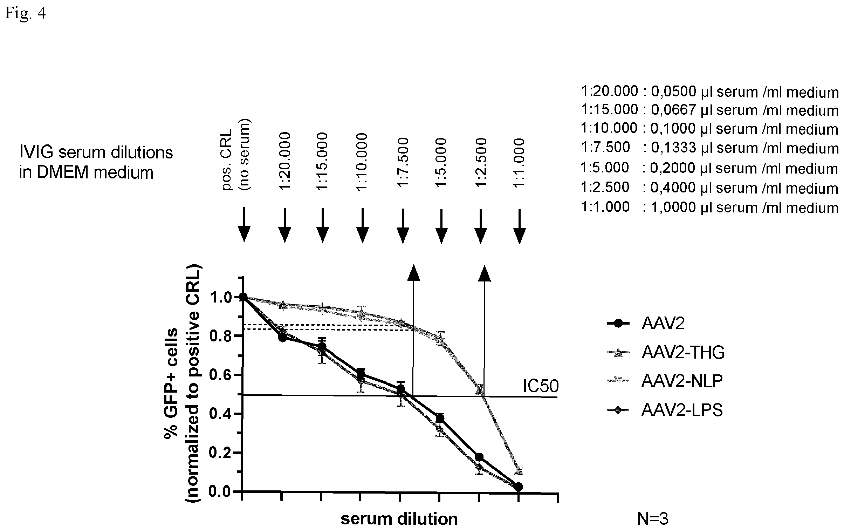

Fig. 4 shows the FACS results, wherein the percentage of EGFP-positive cells was normalized to the percentage determined for the positive control (pos. CRL) =1. It is found that the wild-type AAV2 (AAV2) and the comparative viral vector particle containing CAP with SEQ ID NO: 62 as the inserted amino acid section (AAV2-LPS) are neutralized already at higher dilutions of the IVIG serum than viral vector particles of the invention, e.g. containing the inserted amino acid section of SEQ ID NO: 1 (AAV2-THG) or containing the inserted amino acid section of SEQ ID NO: 11 (AAV2-NLP).

The production of viral vector particles according to the invention was performed as described herein for the expression and vector copy number analysis in mouse tissue.

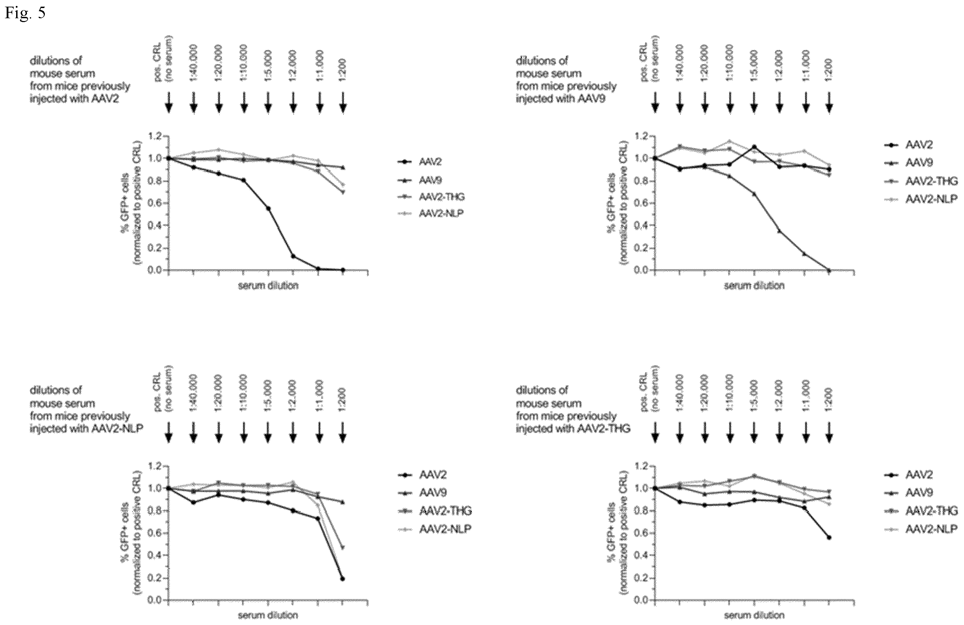

Fig. 5 shows the FACS results, wherein the percentage of EGFP-positive cells was normalized to the percentage determined for the positive control (pos. CRL) =1. It is found that mouse serum from mice which were previously injected with wild-type AAV9 (AAV9) only neutralise AAV9, whereas AAV2 and the viral vector particles of the invention transduce cells despite the presence of this serum. Similar, mouse serum from mice which were previously injected with wild-type AAV2 (AAV2) only neutralise AAV2, whereas AAV9 and the viral vector particles of the invention remain their ability to transduce cell. Of note, serum of mice previously injected with AAV-NLP shows poor neutralisation capacity, resulting in neutralisation of AAV2 and the viral vector particles of the invention only at low serum dilutions. Similar, serum of mice previously injected with AAV-THG shows poor neutralisation capacity, resulting in weak neutralisation of only wild-type AAV2 at low serum dilutions.

## Claims

1. AAV2 viral vector particle comprising a nucleic acid construct for an effector molecule, **characterized by** comprising a capsid protein (CAP) which C-terminally to amino acid No. 587 or No. 588 or No. 453 of the wild-type amino acid sequence of CAP of SEQ ID NO: 56 contains an inserted amino acid section comprising one of the amino acid sequences selected from SEQ ID NO: 1 to SEQ ID NO: 53.

2. AAV2 viral vector particle according to claim 1, **characterized in that** a linker sequence of 1 to 4 amino acids is arranged between amino acid No. 587 or No. 588 or No. 453 of the N-terminal section of CAP and the N-terminal amino acid of the inserted amino acid section.

3. AAV2 viral vector particle according to one of the preceding claims, **characterized in that** a linker sequence of 1 to 3 amino acids is arranged between the C-terminus of the inserted amino acid section and the N-terminal amino acid of the remaining C-terminal portion of CAP.

4. AAV2 viral vector particle according to one of the preceding claims, **characterized in that** the remaining C-terminal portion of CAP are amino acids 588 to 735, or amino acids 589 to 735, or amino acids 454 to 735, of SEQ ID NO: 56.

5. AAV2 viral vector particle according to one of the preceding claims, **characterized by** the mutations R585A (amino acid 585 Arg to Ala) and R588A (amino acid 588 Arg to Ala).

6. AAV2 viral vector particle according to one of the preceding claims for use in the treatment of a disease or defect of cardiac myocytes, or in the treatment of a disease or defect of muscular myocytes or of skeletal muscle cells.

7. AAV2 viral vector particle for use in the treatment of a disease or defect of cardiomyocytes according to claim 6, **characterized in that** the vector particle contains a nucleic acid construct comprising an effector sequence.

8. AAV2 viral vector particle for use in the treatment of a disease or defect of cardiomyocytes according to claim 6 or 7, **characterized in that** the effector sequence is an expression cassette encoding an effector molecule.

9. AAV2 viral vector particle for use in the treatment of a disease or defect of cardiomyocytes according to one of claims 6 to 8, wherein the disease or defect is cardiac hypertrophy, myocardial infarction, cardiotoxicity or cardiac failure.

10. AAV2 vector particle for use in the treatment of a disease or defect of cardiomyocytes according to one of claims 6 to 9, wherein the treatment is in vivo or ex vivo transduction of cardiomyocytes.

11. AAV2 viral vector particle for use in the treatment of a disease or defect of cardiomyocytes according to one of claims 6 to 10, wherein the person receiving the viral vector particle has antibody neutralizing wild-type AAV2 and/or has antibody neutralizing AAV9.

12. Process for producing AAV2 viral vector particles by delivery of components for AAV vector production in a cultivated eukaryotic cell, followed by cell lysis and removal of cellular components and plasmid DNA, and further purification of AAV viral vector particles, **characterized in that** the AAV2 viral vector particle comprises a capsid protein (CAP) which C-terminally to amino acid No. 587 or C-terminally to amino acid No. 588 or C-terminally to amino acid No. 453 of the wild-type amino acid sequence of CAP contains an inserted amino acid section comprising one of the amino acid sequences selected from SEQ ID NO: 1 to SEQ ID NO: 53.

**Figures**

Fig. 1

initial library:
~ 4x10$^6$ different nt sequences at the 587 insert position

1. gene:
   1. selection: *rep*
   2./3. selection: *EGFP*

additional required plasmids for AAV library preparation

ITR — 1. *gene* — *cap* — ITR

587 — NotI — NNB NNB NNB NNB NNB NNB NNB — AscI — 588

N A A A **X X X X X X X** A A R

cloning

**X X X X X X X**
NotI — AscI

PCR amplification of variable DNA insert

Transfection

HEK293 cells

packaging of AAV particles

iodixanol gradient purification

3 selection rounds

DNA Extraction

AAV library

AAV injection

day 0
TAC surgery

8 weeks of TAC

TAC confirmed by echocardiography

isolation of desired target cells

target cells

C57BL/6N
6-8 weeks old

EP 3 868 875 A1

Fig. 2

Fig. 3 A

B

C

Fig. 4

IVIG serum dilutions
in DMEM medium

1:20.000 : 0,0500 µl serum /ml medium
1:15.000 : 0,0667 µl serum /ml medium
1:10.000 : 0,1000 µl serum /ml medium
1:7.500   : 0,1333 µl serum /ml medium
1:5.000   : 0,2000 µl serum /ml medium
1:2.500   : 0,4000 µl serum /ml medium
1:1.000   : 1,0000 µl serum /ml medium

AAV2
AAV2-THG
AAV2-NLP
AAV2-LPS

N=3

Fig. 5

Europäisches
Patentamt

European
Patent Office

Office européen
des brevets

# EUROPEAN SEARCH REPORT

Application Number

EP 20 15 8633

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | Y YING ET AL: "Heart-targeted adeno-associated viral vectors selected by in vivo biopanning of a random viral display peptide library", GENE THERAPY, vol. 17, no. 8, 15 April 2010 (2010-04-15), pages 980-990, XP055147105, ISSN: 0969-7128, DOI: 10.1038/gt.2010.44 * the whole document * | 1-12 | INV. C12N7/00 C12N15/864 C07K14/005 |
| A | L. YANG ET AL: "A myocardium tropic adeno-associated virus (AAV) evolved by DNA shuffling and in vivo selection", PROCEEDINGS OF THE NATIONAL ACADEMY OF SCIENCES, vol. 106, no. 10, 10 March 2009 (2009-03-10), pages 3946-3951, XP055165570, ISSN: 0027-8424, DOI: 10.1073/pnas.0813207106 * the whole document * | 1-12 | |
| A | WO 2019/076856 A1 (VIGENERON GMBH [DE]) 25 April 2019 (2019-04-25) * the whole document * | 1-12 | TECHNICAL FIELDS SEARCHED (IPC)<br><br>C07K<br>C12N |
| A | WO 2004/083441 A2 (DEUTSCHES KREBSFORSCH [DE]; UNIV ALBERT LUDWIGS FREIBURG [DE] ET AL.) 30 September 2004 (2004-09-30) * the whole document * | 1-12 | |

~~The present search report has been drawn up for all claims~~

| Place of search | Date of completion of the search | Examiner |
|---|---|---|
| Munich | 14 July 2020 | Brenz Verca, Stefano |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons
 
& : member of the same patent family, corresponding
document

EPO FORM 1503 03.82 (P04C01)

Europäisches
Patentamt

European
Patent Office

Office européen
des brevets

**Application Number**

EP 20 15 8633

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-12(partially)

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION**
**SHEET B**

The Search Division considers that the present European patent application does not comply with the requirements of unity of invention and relates to several inventions or groups of inventions, namely:

1. claims: 1-12(partially)

AAV2 viral vector particle comprising a nucleic acid construct for an effector molecule, characterized by comprising a capsid protein (CAP) which C-terminally to amino acid No. 587 or No. 588 or No. 453 of the wild-type amino acid sequence of CAP of SEQ ID NO: 56 contains an inserted amino acid section comprising one of the amino acid sequences selected from SEQ ID NO: 1 or SEQ ID NO: 11 ; AAV2 viral vector particle according to one of the preceding claims for use in the treatment of a disease or defect of cardiac myocytes, or in the treatment of a disease or defect of muscular myocytes or of skeletal muscle cells; Process for producing AAV2 viral vector particles by delivery of components for AAV vector production in a cultivated eukaryotic cell, followed by cell lysis and removal of cellular components and plasmid DNA, and further purification of AAV viral vector particles, characterized in that the AAV2 viral vector particle comprises a capsid protein (CAP) which C-terminally to amino acid No. 587 or C-terminally to amino acid No. 588 or C-terminally to amino acid No. 453 of the wild-type amino acid sequence of CAP contains an inserted amino acid section comprising one of the amino acid sequences selected from SEQ ID NO: 1 and SEQ ID NO: 11.

---

2-52. claims: 1-12(partially)

The same subject matter of invention 1, however, with the difference that each of the sequence identifiers for a peptide of the following list represents a separate invention:
Invention 2 : SEQ ID No:2
Invention 3 : SEQ ID No:3
Invention 4 : SEQ ID No:4
Invention 5 : SEQ ID No:5
Invention 6 : SEQ ID No:6
Invention 7 : SEQ ID No:7
Invention 8 : SEQ ID No:8
Invention 9 : SEQ ID No:9
Invention 10 : SEQ ID No:10
Invention 11 : SEQ ID No:12
Invention 12 : SEQ ID No:13
Invention 13 : SEQ ID No:14
Invention 14 : SEQ ID No:15
Invention 15 : SEQ ID No:16
Invention 16 : SEQ ID No:17
Invention 17 : SEQ ID No:18
Invention 18 : SEQ ID No:19
Invention 19 : SEQ ID No:20

page 1 of 2

Europäisches
Patentamt

European
Patent Office

Office européen
des brevets

**LACK OF UNITY OF INVENTION
SHEET B**

The Search Division considers that the present European patent application does not comply with the requirements of unity of invention and relates to several inventions or groups of inventions, namely:

```
Invention 20 : SEQ ID No:21
Invention 21 : SEQ ID No:22
Invention 22 : SEQ ID No:23
Invention 23 : SEQ ID No:24
Invention 24 : SEQ ID No:25
Invention 25 : SEQ ID No:26
Invention 26 : SEQ ID No:27
Invention 27 : SEQ ID No:28
Invention 28 : SEQ ID No:29
Invention 29 : SEQ ID No:30
Invention 30 : SEQ ID No:31
Invention 31 : SEQ ID No:32
Invention 32 : SEQ ID No:33
Invention 33 : SEQ ID No:34
Invention 34 : SEQ ID No:35
Invention 35 : SEQ ID No:36
Invention 36 : SEQ ID No:37
Invention 37 : SEQ ID No:38
Invention 38 : SEQ ID No:39
Invention 39 : SEQ ID No:40
Invention 40 : SEQ ID No:41
Invention 41 : SEQ ID No:42
Invention 42 : SEQ ID No:43
Invention 43 : SEQ ID No:44
Invention 44 : SEQ ID No:45
Invention 45 : SEQ ID No:46
Invention 46 : SEQ ID No:47
Invention 47 : SEQ ID No:48
Invention 48 : SEQ ID No:49
Invention 49 : SEQ ID No:50
Invention 50 : SEQ ID No:51
Invention 51 : SEQ ID No:52
Invention 52 : SEQ ID No:53
                 ---
```

page 2 of 2

## ANNEX TO THE EUROPEAN SEARCH REPORT
## ON EUROPEAN PATENT APPLICATION NO.

EP 20 15 8633

This annex lists the patent family members relating to the patent documents cited in the above-mentioned European search report.
The members are as contained in the European Patent Office EDP file on
The European Patent Office is in no way liable for these particulars which are merely given for the purpose of information.

14-07-2020

| Patent document cited in search report | | Publication date | Patent family member(s) | | Publication date |
|---|---|---|---|---|---|
| WO 2019076856 | A1 | 25-04-2019 | AU | 2018350700 A1 | 07-05-2020 |
| | | | CA | 3078277 A1 | 25-04-2019 |
| | | | CN | 111601884 A | 28-08-2020 |
| | | | EP | 3697896 A1 | 26-08-2020 |
| | | | KR | 20200098481 A | 20-08-2020 |
| | | | US | 2020308553 A1 | 01-10-2020 |
| | | | WO | 2019076856 A1 | 25-04-2019 |
| WO 2004083441 | A2 | 30-09-2004 | US | 2007172460 A1 | 26-07-2007 |
| | | | WO | 2004083441 A2 | 30-09-2004 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **PERABO et al.** *Molecular Therapy,* 2003, vol. 8 (1), 151-157
- **YING et al.** *Gene Therapy,* 2010, vol. 17, 980-990
- **WANG et al.** *Nature Reviews Drug Discovery,* 2019, 358-378
- **ROCKMAN et al.** *PNAS,* 1991, vol. 88, 8277-8281

- **ZHANG et al.** *Hum. Gene Ther.,* 2019, 1284-1296
- **ZINCARELLI et al.** *Molecular Therapy,* 2008, vol. 16 (6), 1073-1080
- **HAJJAR ; ISHIKAWA.** *Circulation Res,* 2017, vol. 120 (1), 33-35

SEQUENCE LISTING

<110> Medizinische Hochschule Hannover

<120> Viral particle based on AAV2 for gene therapy

<130> M1091EP

<160> 62

<170> BiSSAP 1.3.6

<210> 1
<211> 7
<212> PRT
<213> Artificial Sequence


<220>
<223> inserted amino acid section

<400> 1
Thr His Gly Thr Pro Ala Asp
1               5

<210> 2
<211> 7
<212> PRT
<213> Artificial Sequence


<220>
<223> inserted amino acid section

<400> 2
Gly Cys Gly Gly Ile Pro Glu
1               5

<210> 3
<211> 7
<212> PRT
<213> Artificial Sequence


<220>
<223> inserted amino acid section

<400> 3
Gln His Glu Ala Leu Arg Cys
1               5

<210> 4
<211> 7
<212> PRT
<213> Artificial Sequence


<220>
<223> inserted amino acid section

<400> 4
Asp Val Pro Thr Thr Gly Ile
1               5

```
<210> 5
<211> 7
<212> PRT
<213> Artificial Sequence


<220>
<223> inserted amino acid section

<400> 5
Asp Thr Arg Cys Pro Ser Ser
1               5


<210> 6
<211> 7
<212> PRT
<213> Artificial Sequence


<220>
<223> inserted amino acid section

<400> 6
Ala Leu Leu Cys Arg His Asp
1               5


<210> 7
<211> 7
<212> PRT
<213> Artificial Sequence


<220>
<223> inserted amino acid section

<400> 7
Gly Ser Leu Gln Ser Gly Glu
1               5


<210> 8
<211> 7
<212> PRT
<213> Artificial Sequence


<220>
<223> inserted amino acid section

<400> 8
Asp Pro Pro Ser Ser Ser Ala
1               5


<210> 9
<211> 7
<212> PRT
<213> Artificial Sequence


<220>
<223> inserted amino acid section

<400> 9
```

```
Val Ser Ser Thr Ser Pro Arg
1               5


<210> 10
<211> 7
<212> PRT
<213> Artificial Sequence


<220>
<223> inserted amino acid section

<400> 10
Val Ser Ser Thr Pro Pro Arg
1               5


<210> 11
<211> 7
<212> PRT
<213> Artificial Sequence


<220>
<223> inserted amino acid section

<400> 11
Asn Leu Pro Gly Ser Gly Asp
1               5


<210> 12
<211> 7
<212> PRT
<213> Artificial Sequence


<220>
<223> inserted amino acid section

<400> 12
Ala Pro Ser Glu Ser Pro Asn
1               5


<210> 13
<211> 7
<212> PRT
<213> Artificial Sequence


<220>
<223> inserted amino acid section

<400> 13
Gly Ile Glu Ile Gly Cys Ser
1               5


<210> 14
<211> 7
<212> PRT
<213> Artificial Sequence


<220>
<223> inserted amino acid section
```

```
<400> 14
Val Gly Pro Ser Arg Gly Ser
1               5


<210> 15
<211> 7
<212> PRT
<213> Artificial Sequence


<220>
<223> inserted amino acid section

<400> 15
Ser Asn Gly Asn Ala Cys Gly
1               5


<210> 16
<211> 7
<212> PRT
<213> Artificial Sequence


<220>
<223> inserted amino acid section

<400> 16
Ala Glu Arg Gln Pro Thr Gly
1               5


<210> 17
<211> 7
<212> PRT
<213> Artificial Sequence


<220>
<223> inserted amino acid section

<400> 17
Gly Gly Glu Leu Asp Cys Arg
1               5


<210> 18
<211> 7
<212> PRT
<213> Artificial Sequence


<220>
<223> inserted amino acid section

<400> 18
Ala Asn Tyr Ser Pro Pro Ala
1               5


<210> 19
<211> 7
<212> PRT
<213> Artificial Sequence
```

```
<220>
<223> inserted amino acid section

<400> 19
Thr Gly Ser Pro Cys Thr Ala
1               5


<210> 20
<211> 7
<212> PRT
<213> Artificial Sequence



<220>
<223> inserted amino acid section

<400> 20
Met Arg Thr Gly Val Ser Val
1               5


<210> 21
<211> 7
<212> PRT
<213> Artificial Sequence



<220>
<223> inserted amino acid section

<400> 21
Ser Asp Trp Thr Glu Asp Pro
1               5


<210> 22
<211> 7
<212> PRT
<213> Artificial Sequence



<220>
<223> inserted amino acid section

<400> 22
Gly Ser Pro Gly Asp Ala Gly
1               5


<210> 23
<211> 7
<212> PRT
<213> Artificial Sequence



<220>
<223> inserted amino acid section

<400> 23
Gln Ser Ala Gly Leu Glu Cys
1               5


<210> 24
<211> 7
<212> PRT
<213> Artificial Sequence
```

```
<220>
<223> inserted amino acid section

<400> 24
Ser Gln Asn Ser Thr Ser Arg
1               5


<210> 25
<211> 7
<212> PRT
<213> Artificial Sequence


<220>
<223> inserted amino acid section

<400> 25
Cys Pro Tyr Gln Pro Pro Gly
1               5


<210> 26
<211> 7
<212> PRT
<213> Artificial Sequence


<220>
<223> inserted amino acid section

<400> 26
Ala Arg Asp Ser Gly His Thr
1               5


<210> 27
<211> 7
<212> PRT
<213> Artificial Sequence


<220>
<223> inserted amino acid section

<400> 27
Tyr Pro Pro Pro Cys Glu Ser
1               5


<210> 28
<211> 7
<212> PRT
<213> Artificial Sequence


<220>
<223> inserted amino acid section

<400> 28
Ser Pro Gly Gln Ser Cys Trp
1               5


<210> 29
<211> 7
```

```
<212> PRT
<213> Artificial Sequence


<220>
<223> inserted amino acid section

<400> 29
Gly Asn Gly Ala Gly Ala His
1               5

<210> 30
<211> 7
<212> PRT
<213> Artificial Sequence


<220>
<223> inserted amino acid section

<400> 30
Gly Cys Ala Gly Gly Asn Tyr
1               5

<210> 31
<211> 7
<212> PRT
<213> Artificial Sequence


<220>
<223> inserted amino acid section

<400> 31
Val Gly Ser Thr Leu Pro Gln
1               5

<210> 32
<211> 7
<212> PRT
<213> Artificial Sequence


<220>
<223> inserted amino acid section

<400> 32
Tyr Gly Ala Arg His Asp Gly
1               5

<210> 33
<211> 7
<212> PRT
<213> Artificial Sequence


<220>
<223> inserted amino acid section

<400> 33
Asp Cys Thr Pro Gly Ala Ser
1               5
```

```
<210> 34
<211> 7
<212> PRT
<213> Artificial Sequence


<220>
<223> inserted amino acid section

<400> 34
Cys Phe Pro Arg Pro Asp Glu
1               5


<210> 35
<211> 7
<212> PRT
<213> Artificial Sequence


<220>
<223> inserted amino acid section

<400> 35
Phe Asp Pro Gly Tyr Arg Ser
1               5


<210> 36
<211> 7
<212> PRT
<213> Artificial Sequence


<220>
<223> inserted amino acid section

<400> 36
Ala Asn His Gly Val Thr Arg
1               5


<210> 37
<211> 7
<212> PRT
<213> Artificial Sequence


<220>
<223> inserted amino acid section

<400> 37
Asp Ser Val Ser Leu Gly Ala
1               5


<210> 38
<211> 7
<212> PRT
<213> Artificial Sequence


<220>
<223> inserted amino acid section

<400> 38
Ala Gly Asn Gln Thr Arg Ser
```

1                    5

<210> 39
<211> 7
<212> PRT
<213> Artificial Sequence


<220>
<223> inserted amino acid section

<400> 39
Gly Val Pro Gln Arg Pro Glu
1                    5

<210> 40
<211> 7
<212> PRT
<213> Artificial Sequence


<220>
<223> inserted amino acid section

<400> 40
Glu Pro Ser Gly Ser Val Cys
1                    5

<210> 41
<211> 7
<212> PRT
<213> Artificial Sequence


<220>
<223> inserted amino acid section

<400> 41
Glu Leu His Ser Pro Ser Ala
1                    5

<210> 42
<211> 7
<212> PRT
<213> Artificial Sequence


<220>
<223> inserted amino acid section

<400> 42
Pro Asn Glu Gly Ala Gly Arg
1                    5

<210> 43
<211> 7
<212> PRT
<213> Artificial Sequence


<220>
<223> inserted amino acid section

```
<400> 43
Ser Gly Ser Pro Thr His Cys
1               5


<210> 44
<211> 7
<212> PRT
<213> Artificial Sequence



<220>
<223> inserted amino acid section

<400> 44
Met Pro Arg Cys Thr Glu Ala
1               5

<210> 45
<211> 7
<212> PRT
<213> Artificial Sequence



<220>
<223> inserted amino acid section

<400> 45
Thr Gly Ser Pro Tyr Thr Ala
1               5

<210> 46
<211> 7
<212> PRT
<213> Artificial Sequence



<220>
<223> inserted amino acid section

<400> 46
Asn Leu Thr Arg Pro Ala Leu
1               5

<210> 47
<211> 7
<212> PRT
<213> Artificial Sequence



<220>
<223> inserted amino acid section

<400> 47
Ser Pro Thr Arg Asp Pro Cys
1               5

<210> 48
<211> 7
<212> PRT
<213> Artificial Sequence



<220>
```

<223> inserted amino acid section

<400> 48
Tyr Ala Pro Ala Arg Ser Ser
1               5


<210> 49
<211> 7
<212> PRT
<213> Artificial Sequence


<220>
<223> inserted amino acid section

<400> 49
Val Pro Val Arg Pro Thr Ser
1               5


<210> 50
<211> 7
<212> PRT
<213> Artificial Sequence


<220>
<223> inserted amino acid section

<400> 50
Arg Val Gly His Gly Ser Ala
1               5


<210> 51
<211> 7
<212> PRT
<213> Artificial Sequence


<220>
<223> inserted amino acid section

<400> 51
Arg Ile Ser Thr Glu Gly Ala
1               5


<210> 52
<211> 7
<212> PRT
<213> Artificial Sequence


<220>
<223> inserted amino acid section

<400> 52
Gln Gln Thr Gly Gly Thr Arg
1               5


<210> 53
<211> 7
<212> PRT
<213> Artificial Sequence

```
<220>
<223> inserted amino acid section

<400> 53
Asp Thr Met Pro Ser Gly Val
1               5


<210> 54
<211> 747
<212> PRT
<213> Artificial Sequence


<220>
<221> CHAIN
<222> 1..587
<223> N-terminal section of native CAP

<220>
<223> CAP protein with inserted amino acid section

<220>
<221> CHAIN
<222> 588..590
<223> linker

<220>
<221> CHAIN
<222> 591..597
<223> inserted amino acid sequence

<220>
<221> CHAIN
<222> 598..599
<223> linker

<220>
<221> CHAIN
<222> 600..747
<223> C-terminal section of CAP

<400> 54
Met Ala Ala Asp Gly Tyr Leu Pro Asp Trp Leu Glu Asp Thr Leu Ser
1               5                   10                  15
Glu Gly Ile Arg Gln Trp Trp Lys Leu Lys Pro Gly Pro Pro Pro
                20              25              30
Lys Pro Ala Glu Arg His Lys Asp Asp Ser Arg Gly Leu Val Leu Pro
        35              40                  45
Gly Tyr Lys Tyr Leu Gly Pro Phe Asn Gly Leu Asp Lys Gly Glu Pro
    50              55                  60
Val Asn Glu Ala Asp Ala Ala Leu Glu His Asp Lys Ala Tyr Asp
65              70                  75                  80
Arg Gln Leu Asp Ser Gly Asp Asn Pro Tyr Leu Lys Tyr Asn His Ala
                85              90                  95
Asp Ala Glu Phe Gln Glu Arg Leu Lys Glu Asp Thr Ser Phe Gly Gly
            100             105                 110
Asn Leu Gly Arg Ala Val Phe Gln Ala Lys Lys Arg Val Leu Glu Pro
            115             120                 125
Leu Gly Leu Val Glu Glu Pro Val Lys Thr Ala Pro Gly Lys Lys Arg
            130             135                 140
Pro Val Glu His Ser Pro Val Glu Pro Asp Ser Ser Ser Gly Thr Gly
145             150                 155                 160
```

21

Lys Ala Gly Gln Gln Pro Ala Arg Lys Arg Leu Asn Phe Gly Gln Thr
165              170                  175

Gly Asp Ala Asp Ser Val Pro Asp Pro Gln Pro Leu Gly Gln Pro Pro
         180              185                  190

Ala Ala Pro Ser Gly Leu Gly Thr Asn Thr Met Ala Thr Gly Ser Gly
         195              200                  205

Ala Pro Met Ala Asp Asn Asn Glu Gly Ala Asp Gly Val Gly Asn Ser
210              215                  220

Ser Gly Asn Trp His Cys Asp Ser Thr Trp Met Gly Asp Arg Val Ile
225              230                  235                      240

Thr Thr Ser Thr Arg Thr Trp Ala Leu Pro Thr Tyr Asn Asn His Leu
             245              250                      255

Tyr Lys Gln Ile Ser Ser Gln Ser Gly Ala Ser Asn Asp Asn His Tyr
             260              265                      270

Phe Gly Tyr Ser Thr Pro Trp Gly Tyr Phe Asp Phe Asn Arg Phe His
         275              280                      285

Cys His Phe Ser Pro Arg Asp Trp Gln Arg Leu Ile Asn Asn Asn Trp
290              295                  300

Gly Phe Arg Pro Lys Arg Leu Asn Phe Lys Leu Phe Asn Ile Gln Val
305              310                  315                      320

Lys Glu Val Thr Gln Asn Asp Gly Thr Thr Ile Ala Asn Asn Leu
             325              330                      335

Thr Ser Thr Val Gln Val Phe Thr Asp Ser Glu Tyr Gln Leu Pro Tyr
             340              345                      350

Val Leu Gly Ser Ala His Gln Gly Cys Leu Pro Pro Phe Pro Ala Asp
         355              360                  365

Val Phe Met Val Pro Gln Tyr Gly Tyr Leu Thr Leu Asn Asn Gly Ser
370              375                  380

Gln Ala Val Gly Arg Ser Ser Phe Tyr Cys Leu Glu Tyr Phe Pro Ser
385              390                  395                      400

Gln Met Leu Arg Thr Gly Asn Asn Phe Thr Phe Ser Tyr Thr Phe Glu
             405              410                      415

Asp Val Pro Phe His Ser Ser Tyr Ala His Ser Gln Ser Leu Asp Arg
         420              425                  430

Leu Met Asn Pro Leu Ile Asp Gln Tyr Leu Tyr Tyr Leu Ser Arg Thr
         435              440                  445

Asn Thr Pro Ser Gly Thr Thr Thr Gln Ser Arg Leu Gln Phe Ser Gln
         450              455                  460

Ala Gly Ala Ser Asp Ile Arg Asp Gln Ser Arg Asn Trp Leu Pro Gly
465              470                  475                      480

Pro Cys Tyr Arg Gln Gln Arg Val Ser Lys Thr Ser Ala Asp Asn Asn
             485              490                      495

Asn Ser Glu Tyr Ser Trp Thr Gly Ala Thr Lys Tyr His Leu Asn Gly
             500              505                      510

Arg Asp Ser Leu Val Asn Pro Gly Pro Ala Met Ala Ser His Lys Asp
         515              520                  525

Asp Glu Glu Lys Phe Phe Pro Gln Ser Gly Val Leu Ile Phe Gly Lys
         530              535                  540

Gln Gly Ser Glu Lys Thr Asn Val Asp Ile Glu Lys Val Met Ile Thr
545              550                  555                      560

Asp Glu Glu Glu Ile Arg Thr Thr Asn Pro Val Ala Thr Glu Gln Tyr
             565              570                      575

Gly Ser Val Ser Thr Asn Leu Gln Arg Gly Asn Ala Ser Ala Thr His
         580              585                  590

Gly Thr Pro Ala Asp Ala Ala Arg Gln Ala Ala Thr Ala Asp Val Asn
         595              600                  605

Thr Gln Gly Val Leu Pro Gly Met Val Trp Gln Asp Arg Asp Val Tyr
610              615                  620

Leu Gln Gly Pro Ile Trp Ala Lys Ile Pro His Thr Asp Gly His Phe
625              630                  635                      640

His Pro Ser Pro Leu Met Gly Gly Phe Gly Leu Lys His Pro Pro Pro
             645              650                      655

Gln Ile Leu Ile Lys Asn Thr Pro Val Pro Ala Asn Pro Ser Thr Thr

```
                    660                    665                    670
        Phe Ser Ala Ala Lys Phe Ala Ser Phe Ile Thr Gln Tyr Ser Thr Gly
                    675                    680                    685
        Gln Val Ser Val Glu Ile Glu Trp Glu Leu Gln Lys Glu Asn Ser Lys
                    690                    695                    700
        Arg Trp Asn Pro Glu Ile Gln Tyr Thr Ser Asn Tyr Asn Lys Ser Val
        705                    710                    715                    720
        Asn Val Asp Phe Thr Val Asp Thr Asn Gly Val Tyr Ser Glu Pro Arg
                    725                    730                    735
        Pro Ile Gly Thr Arg Tyr Leu Thr Arg Asn Leu
                    740                    745
```

```
<210> 55
<211> 747
<212> PRT
<213> Artificial Sequence


<220>
<221> CHAIN
<222> 1..587
<223> N-terminal section of native CAP

<220>
<223> CAP with inserted amino acid section

<220>
<221> CHAIN
<222> 588..590
<223> linker

<220>
<221> CHAIN
<222> 591..597
<223> inserted amino acid sequence

<220>
<221> CHAIN
<222> 598..599
<223> linker

<220>
<221> CHAIN
<222> 600..747
<223> C-terminal section of CAP

<400> 55
Met Ala Ala Asp Gly Tyr Leu Pro Asp Trp Leu Glu Asp Thr Leu Ser
1                   5                   10                  15
Glu Gly Ile Arg Gln Trp Trp Lys Leu Lys Pro Gly Pro Pro Pro Pro
            20                  25                  30
Lys Pro Ala Glu Arg His Lys Asp Asp Ser Arg Gly Leu Val Leu Pro
            35                  40                  45
Gly Tyr Lys Tyr Leu Gly Pro Phe Asn Gly Leu Asp Lys Gly Glu Pro
            50                  55                  60
Val Asn Glu Ala Asp Ala Ala Ala Leu Glu His Asp Lys Ala Tyr Asp
65                  70                  75                  80
Arg Gln Leu Asp Ser Gly Asp Asn Pro Tyr Leu Lys Tyr Asn His Ala
                85                  90                  95
Asp Ala Glu Phe Gln Glu Arg Leu Lys Glu Asp Thr Ser Phe Gly Gly
            100                 105                 110
Asn Leu Gly Arg Ala Val Phe Gln Ala Lys Lys Arg Val Leu Glu Pro
            115                 120                 125
```

23

```
Leu Gly Leu Val Glu Glu Pro Val Lys Thr Ala Pro Gly Lys Lys Arg
    130                 135                 140
Pro Val Glu His Ser Pro Val Glu Pro Asp Ser Ser Ser Gly Thr Gly
145                 150                 155                 160
Lys Ala Gly Gln Gln Pro Ala Arg Lys Arg Leu Asn Phe Gly Gln Thr
                165                 170                 175
Gly Asp Ala Asp Ser Val Pro Asp Pro Gln Pro Leu Gly Gln Pro Pro
            180                 185                 190
Ala Ala Pro Ser Gly Leu Gly Thr Asn Thr Met Ala Thr Gly Ser Gly
        195                 200                 205
Ala Pro Met Ala Asp Asn Asn Glu Gly Ala Asp Gly Val Gly Asn Ser
    210                 215                 220
Ser Gly Asn Trp His Cys Asp Ser Thr Trp Met Gly Asp Arg Val Ile
225                 230                 235                 240
Thr Thr Ser Thr Arg Thr Trp Ala Leu Pro Thr Tyr Asn Asn His Leu
                245                 250                 255
Tyr Lys Gln Ile Ser Ser Gln Ser Gly Ala Ser Asn Asp Asn His Tyr
            260                 265                 270
Phe Gly Tyr Ser Thr Pro Trp Gly Tyr Phe Asp Phe Asn Arg Phe His
        275                 280                 285
Cys His Phe Ser Pro Arg Asp Trp Gln Arg Leu Ile Asn Asn Asn Trp
    290                 295                 300
Gly Phe Arg Pro Lys Arg Leu Asn Phe Lys Leu Phe Asn Ile Gln Val
305                 310                 315                 320
Lys Glu Val Thr Gln Asn Asp Gly Thr Thr Thr Ile Ala Asn Asn Leu
                325                 330                 335
Thr Ser Thr Val Gln Val Phe Thr Asp Ser Glu Tyr Gln Leu Pro Tyr
            340                 345                 350
Val Leu Gly Ser Ala His Gln Gly Cys Leu Pro Pro Phe Pro Ala Asp
        355                 360                 365
Val Phe Met Val Pro Gln Tyr Gly Tyr Leu Thr Leu Asn Asn Gly Ser
    370                 375                 380
Gln Ala Val Gly Arg Ser Ser Phe Tyr Cys Leu Glu Tyr Phe Pro Ser
385                 390                 395                 400
Gln Met Leu Arg Thr Gly Asn Asn Phe Thr Phe Ser Tyr Thr Phe Glu
                405                 410                 415
Asp Val Pro Phe His Ser Ser Tyr Ala His Ser Gln Ser Leu Asp Arg
            420                 425                 430
Leu Met Asn Pro Leu Ile Asp Gln Tyr Leu Tyr Tyr Leu Ser Arg Thr
        435                 440                 445
Asn Thr Pro Ser Gly Thr Thr Thr Gln Ser Arg Leu Gln Phe Ser Gln
    450                 455                 460
Ala Gly Ala Ser Asp Ile Arg Asp Gln Ser Arg Asn Trp Leu Pro Gly
465                 470                 475                 480
Pro Cys Tyr Arg Gln Gln Arg Val Ser Lys Thr Ser Ala Asp Asn Asn
                485                 490                 495
Asn Ser Glu Tyr Ser Trp Thr Gly Ala Thr Lys Tyr His Leu Asn Gly
            500                 505                 510
Arg Asp Ser Leu Val Asn Pro Gly Pro Ala Met Ala Ser His Lys Asp
        515                 520                 525
Asp Glu Glu Lys Phe Phe Pro Gln Ser Gly Val Leu Ile Phe Gly Lys
    530                 535                 540
Gln Gly Ser Glu Lys Thr Asn Val Asp Ile Glu Lys Val Met Ile Thr
545                 550                 555                 560
Asp Glu Glu Glu Ile Arg Thr Thr Asn Pro Val Ala Thr Glu Gln Tyr
                565                 570                 575
Gly Ser Val Ser Thr Asn Leu Gln Arg Gly Asn Ala Ser Ala Asn Leu
            580                 585                 590
Pro Gly Ser Gly Asp Ala Ala Arg Gln Ala Ala Thr Ala Asp Val Asn
        595                 600                 605
Thr Gln Gly Val Leu Pro Gly Met Val Trp Gln Asp Arg Asp Val Tyr
    610                 615                 620
Leu Gln Gly Pro Ile Trp Ala Lys Ile Pro His Thr Asp Gly His Phe
```

```
            625                    630                    635                    640
His Pro Ser Pro Leu Met Gly Gly Phe Gly Leu Lys His Pro Pro Pro
                645                    650                    655
Gln Ile Leu Ile Lys Asn Thr Pro Val Pro Ala Asn Pro Ser Thr Thr
                660                    665                    670
Phe Ser Ala Ala Lys Phe Ala Ser Phe Ile Thr Gln Tyr Ser Thr Gly
                675                    680                    685
Gln Val Ser Val Glu Ile Glu Trp Glu Leu Gln Lys Glu Asn Ser Lys
                690                    695                    700
Arg Trp Asn Pro Glu Ile Gln Tyr Thr Ser Asn Tyr Asn Lys Ser Val
705                    710                    715                    720
Asn Val Asp Phe Thr Val Asp Thr Asn Gly Val Tyr Ser Glu Pro Arg
                725                    730                    735
Pro Ile Gly Thr Arg Tyr Leu Thr Arg Asn Leu
                740                    745
```

<210> 56
<211> 735
<212> PRT
<213> Adeno-associated virus - 2


<220>
<223> wild-type CAP protein

<400> 56
```
Met Ala Ala Asp Gly Tyr Leu Pro Asp Trp Leu Glu Asp Thr Leu Ser
1                5                    10                   15
Glu Gly Ile Arg Gln Trp Trp Lys Leu Lys Pro Gly Pro Pro Pro Pro
                20                   25                   30
Lys Pro Ala Glu Arg His Lys Asp Asp Ser Arg Gly Leu Val Leu Pro
                35                   40                   45
Gly Tyr Lys Tyr Leu Gly Pro Phe Asn Gly Leu Asp Lys Gly Glu Pro
                50                   55                   60
Val Asn Glu Ala Asp Ala Ala Ala Leu Glu His Asp Lys Ala Tyr Asp
65                   70                   75                   80
Arg Gln Leu Asp Ser Gly Asp Asn Pro Tyr Leu Lys Tyr Asn His Ala
                85                   90                   95
Asp Ala Glu Phe Gln Glu Arg Leu Lys Glu Asp Thr Ser Phe Gly Gly
                100                  105                  110
Asn Leu Gly Arg Ala Val Phe Gln Ala Lys Lys Arg Val Leu Glu Pro
                115                  120                  125
Leu Gly Leu Val Glu Glu Pro Val Lys Thr Ala Pro Gly Lys Lys Arg
                130                  135                  140
Pro Val Glu His Ser Pro Val Glu Pro Asp Ser Ser Ser Gly Thr Gly
145                  150                  155                  160
Lys Ala Gly Gln Gln Pro Ala Arg Lys Arg Leu Asn Phe Gly Gln Thr
                165                  170                  175
Gly Asp Ala Asp Ser Val Pro Asp Pro Gln Pro Leu Gly Gln Pro Pro
                180                  185                  190
Ala Ala Pro Ser Gly Leu Gly Thr Asn Thr Met Ala Thr Gly Ser Gly
                195                  200                  205
Ala Pro Met Ala Asp Asn Asn Glu Gly Ala Asp Gly Val Gly Asn Ser
210                  215                  220
Ser Gly Asn Trp His Cys Asp Ser Thr Trp Met Gly Asp Arg Val Ile
225                  230                  235                  240
Thr Thr Ser Thr Arg Thr Trp Ala Leu Pro Thr Tyr Asn Asn His Leu
                245                  250                  255
Tyr Lys Gln Ile Ser Ser Gln Ser Gly Ala Ser Asn Asp Asn His Tyr
                260                  265                  270
Phe Gly Tyr Ser Thr Pro Trp Gly Tyr Phe Asp Phe Asn Arg Phe His
                275                  280                  285
Cys His Phe Ser Pro Arg Asp Trp Gln Arg Leu Ile Asn Asn Asn Trp
```

```
                290                      295                        300
Gly Phe Arg Pro Lys Arg Leu Asn Phe Lys Leu Phe Asn Ile Gln Val
305                      310                      315                        320
Lys Glu Val Thr Gln Asn Asp Gly Thr Thr Thr Ile Ala Asn Asn Leu
                325                      330                        335
Thr Ser Thr Val Gln Val Phe Thr Asp Ser Glu Tyr Gln Leu Pro Tyr
                340                      345                        350
Val Leu Gly Ser Ala His Gln Gly Cys Leu Pro Pro Phe Pro Ala Asp
                355                      360                        365
Val Phe Met Val Pro Gln Tyr Gly Tyr Leu Thr Leu Asn Asn Gly Ser
                370                      375                        380
Gln Ala Val Gly Arg Ser Ser Phe Tyr Cys Leu Glu Tyr Phe Pro Ser
385                      390                      395                        400
Gln Met Leu Arg Thr Gly Asn Asn Phe Thr Phe Ser Tyr Thr Phe Glu
                405                      410                        415
Asp Val Pro Phe His Ser Ser Tyr Ala His Ser Gln Ser Leu Asp Arg
                420                      425                        430
Leu Met Asn Pro Leu Ile Asp Gln Tyr Leu Tyr Tyr Leu Ser Arg Thr
                435                      440                        445
Asn Thr Pro Ser Gly Thr Thr Thr Gln Ser Arg Leu Gln Phe Ser Gln
450                      455                      460
Ala Gly Ala Ser Asp Ile Arg Asp Gln Ser Arg Asn Trp Leu Pro Gly
465                      470                      475                        480
Pro Cys Tyr Arg Gln Gln Arg Val Ser Lys Thr Ser Ala Asp Asn Asn
                485                      490                        495
Asn Ser Glu Tyr Ser Trp Thr Gly Ala Thr Lys Tyr His Leu Asn Gly
                500                      505                        510
Arg Asp Ser Leu Val Asn Pro Gly Pro Ala Met Ala Ser His Lys Asp
                515                      520                        525
Asp Glu Glu Lys Phe Phe Pro Gln Ser Gly Val Leu Ile Phe Gly Lys
                530                      535                        540
Gln Gly Ser Glu Lys Thr Asn Val Asp Ile Glu Lys Val Met Ile Thr
545                      550                      555                        560
Asp Glu Glu Glu Ile Arg Thr Thr Asn Pro Val Ala Thr Glu Gln Tyr
                565                      570                        575
Gly Ser Val Ser Thr Asn Leu Gln Arg Gly Asn Arg Gln Ala Ala Thr
                580                      585                        590
Ala Asp Val Asn Thr Gln Gly Val Leu Pro Gly Met Val Trp Gln Asp
                595                      600                        605
Arg Asp Val Tyr Leu Gln Gly Pro Ile Trp Ala Lys Ile Pro His Thr
                610                      615                        620
Asp Gly His Phe His Pro Ser Pro Leu Met Gly Gly Phe Gly Leu Lys
625                      630                      635                        640
His Pro Pro Pro Gln Ile Leu Ile Lys Asn Thr Pro Val Pro Ala Asn
                645                      650                        655
Pro Ser Thr Thr Phe Ser Ala Ala Lys Phe Ala Ser Phe Ile Thr Gln
                660                      665                        670
Tyr Ser Thr Gly Gln Val Ser Val Glu Ile Glu Trp Glu Leu Gln Lys
                675                      680                        685
Glu Asn Ser Lys Arg Trp Asn Pro Glu Ile Gln Tyr Thr Ser Asn Tyr
                690                      695                        700
Asn Lys Ser Val Asn Val Asp Phe Thr Val Asp Thr Asn Gly Val Tyr
705                      710                      715                        720
Ser Glu Pro Arg Pro Ile Gly Thr Arg Tyr Leu Thr Arg Asn Leu
                725                      730                        735
```

<210> 57
<211> 18
<212> DNA
<213> Artificial Sequence

<220>

<223> forward primer in CAP gene

<400> 57
ggtacgacga cgattgcc                                                    18


<210> 58
<211> 18
<212> DNA
<213> Artificial Sequence


<220>
<223> reverse primer CAP gene

<400> 58
atgtccgtcc gtgtgtgg                                                    18


<210> 59
<211> 22
<212> DNA
<213> Artificial Sequence


<220>
<223> Ptbp2 forward primer

<400> 59
tctccattcc ctatgttcat gc                                               22


<210> 60
<211> 22
<212> DNA
<213> Artificial Sequence


<220>
<223> Ptbp2 reverse primer

<400> 60
gttcccgcag aatggtgagg tg                                               22


<210> 61
<211> 20
<212> DNA
<213> Artificial Sequence


<220>
<223> Ptbp2 binding probe

<400> 61
atgttcctcg gaccaacttg                                                  20


<210> 62
<211> 7
<212> PRT
<213> Artificial Sequence

```
<220>
<223> comparative inserted amino acid section

<400> 62
Leu Pro Ser Arg Pro Ser Leu
1               5
```